# FASCICULE DE BREVET EUROPEEN

(11) **EP 2 253 298 B1**
(45) Date de publication et mention de la délivrance du brevet: **30.03.2016**
(21) Numéro de dépôt: 10163069.7
(22) Date de dépôt: 18.05.2010
(51) Int. Cl.: A61J 1/10, A61M 39/10, A61J 1/00, A61M 39/00

(54) **Connecteur pour récipient souple destiné à contenir un liquide médical**
Verbinder für einen flexiblen Behälter zur Aufnahme von medizinischer Flüssigkeit
Connector for a flexible container for receiving a medical liquid

(30) Priorité: 19.05.2009 FR 0953344
(43) Date de publication de la demande: 24.11.2010
(73) Titulaire: Technoflex, 64210 Bidart (FR)
(72) Inventeur: Capitaine, François, 64600, Anglet (FR)
(74) Mandataire: Ipside

(56) Documents cités:
- WO-A1-00/03674
- WO-A1-2008/152871
- WO-A2-03/051270
- US-A- 5 944 709
- US-A1- 2008 208 159

## Description

La présente invention concerne un connecteur pour récipient souple destiné à contenir un liquide médical.

Elle vise encore un récipient souple comprenant au moins un tel connecteur pour l'administration à des fins médicales de substances liquides.

On connaît des connecteurs destinés à être intégrés partiellement dans un récipient souple tel qu'une poche souple pour perfusion ou transfusion, afin d'assurer l'accès au volume intérieur de ce récipient.

Ces connecteurs sont typiquement assemblés aux poches souples par soudure, chacun de ces connecteurs étant alors placé entre les deux parois définissant le volume intérieur de la poche souple correspondante de sorte que le canal interne de ce connecteur est en communication de fluide avec le volume intérieur.

Toutefois, ces connecteurs qui présentent typiquement une section de forme circulaire sont nécessairement écrasés lors de la fabrication des poches souples par soudure.

On a alors constaté que quelque soit le soin apporté à la fabrication de la poche souple, il peut apparaître des micro-fuites du liquide médical contenu dans la poche souple, lesquelles sont liées à une liaison imparfaite au niveau du connecteur et des parois de la poche souple.

Ces pertes peuvent encore être accrues par des manipulations inadéquates du connecteur par un opérateur. A titre purement illustratif, on a observé que la poche pouvait être déchirée au niveau du connecteur lors de manipulations brusques de celui-ci par un opérateur, par exemple, lors d'une ouverture brusque du suremballage protégeant ladite poche souple.

Par ailleurs, on a proposé d'améliorer l'assemblage du connecteur avec les parois de la poche souple en lui conférant une forme non circulaire, voire une forme tubulaire aplatie, au niveau de ses faces de contact avec les parois de la poche souple.

Le document WO 2008/152871 (état de la technique le plus proche) décrit ainsi un connecteur mâle comportant une section d'assemblage destinée à être intégrée dans la poche par soudure à chaud tout en étant pris en sandwich entre les bords de deux feuilles constituant la poche. Le connecteur mâle comporte une plaque de contact disposée entre la section d'assemblage et une partie tubulaire du connecteur, sur la surface périphérique externe de laquelle sont prévues des attaches de contact. Ces attaches sont destinées à former saillie dans une direction radiale par rapport à l'axe central de ce connecteur mâle.

On connaît également par les documents US 5 944 709, WO 00/03674, US 2008/208159 et WO 03/051270 des récipients souples comportant chacun un port d'accès assemblé entre les parois constitutives du récipient correspondant.

Toutefois, ces connecteurs conservent, dans le meilleur des cas, au minimum une ligne de crête générant une ligne d'instabilité lors de leur prise par l'outil de soudure. On peut alors observer que cette ligne d'instabilité occasionne systématiquement un défaut résultant de l'existence d'un léger décalage du connecteur par rapport aux organes presseurs de l'outil de soudage.

La présente invention vise à pallier ces divers inconvénients en proposant un connecteur médical particulièrement simple dans sa conception et dans son mode opératoire, économique et permettant de supprimer les micro-fuites et de limiter au maximum le risque de contamination du contenu d'un récipient sur lequel est monté ce connecteur.

Un autre objectif de la présente invention est un connecteur susceptible d'être adapté à partir d'une base commune par l'adjonction d'une partie fonctionnelle dédiée à une application médicale, ce connecteur pouvant donc présenter différentes fonctionnalités.

Encore un objectif de la présente invention est de permettre à l'opérateur de vérifier très aisément l'intégrité d'une substance active contenue dans un récipient souple comprenant un tel connecteur médical.

A cet effet, l'invention concerne un connecteur pour récipient souple destiné à contenir un liquide médical, ledit connecteur comprenant un conduit interne pour le passage du liquide.

Selon l'invention, ce connecteur comprend :
- une embase destinée à recevoir, à une de ses extrémités, une partie fonctionnelle de ce connecteur,
- ladite embase étant reliée et en communication de fluide à son autre extrémité avec un élément de pincement élastiquement déformable, cet élément élastiquement déformable étant prolongé par un élément de jonction qui peut être intégré dans le récipient souple, l'élément de jonction comportant à chacun de ses bords latéraux une ailette, ces ailettes formant chacune un prolongement de la partie centrale de cet élément de jonction, la partie centrale comprenant sur chacune des faces supérieure et inférieure dudit élément de jonction, une au moins demie coupole placée à l'extrémité de cet élément de jonction et destinée à coopérer avec un organe presseur creux d'un outil de soudage pour assurer un auto-positionnement de l'élément de jonction dans ledit organe presseur

Dans différents modes de réalisation particuliers de ce connecteur, chacun ayant ses avantages particuliers et susceptibles de nombreuses combinaisons techniques possibles:
- l'élément de pincement élastiquement déformable ayant une forme de partie tubulaire avec une section transversale divergeant de la forme tubulaire, les ailettes sont placées dans le prolongement de cette section transversale divergeant de la forme tubulaire,
- ces ailettes comprennent des bords arrondis au moins dans leur partie supérieure en prolongement de la section transversale divergeant de la forme tubulaire,
- ladite une au moins demie coupole est dans le prolongement de la partie tubulaire de l'élément de pincement,
- la partie centrale comprend sur chacune des faces supérieure et inférieure dudit élément de jonction, une coupole,
- l'embase comprend à l'extrémité de sa partie supérieure une ouverture délimitée par une paroi fermée dont la surface externe est destinée à être placée sensiblement au niveau de la surface externe d'un élément de solidarisation de ladite partie fonctionnelle pour permettre leur soudure,

On entend par "sensiblement au niveau de la surface externe d'un élément de jonction de ladite partie fonctionnelle " que la surface externe de la paroi fermée délimitant l'ouverture de l'embase est au même niveau que, ou contiguë à, ou encore affleurant avec, la surface externe de l'élément de solidarisation de ladite partie fonctionnelle aux tolérances d'assemblage près.
- l'embase étant d'un seul tenant, elle comporte dans sa partie supérieure une première portion de conduit interne de diamètre D comportant à son extrémité une ouverture, et dans sa partie inférieure, une deuxième portion de conduit interne reliée à ladite première portion de conduit, ces première et deuxième portions de conduit étant en communication de fluide, la deuxième portion de diamètre d, avec d inférieur à D, formant partiellement saillie à l'intérieur de la première portion de manière à définir un logement entre les parois de ces deux portions pour recevoir l'extrémité de la partie fonctionnelle,
- la première portion comporte un bourrelet continu placé en vis-à vis de ladite deuxième portion en saillie dans ledit logement pour assurer l'étanchéité et la tenue mécanique de l'assemblage entre ladite embase et ladite partie fonctionnelle,
- la partie de la deuxième portion en saillie dans la première portion de l'embase comporte à son extrémité une paroi amincie par un évidement en forme de tronc de cône inversé pour garantir l'étanchéité avec un bec de remplissage destiné à assurer le remplissage du récipient tel qu'une poche souple, sur lequel est monté ce connecteur.

La présente invention permet donc avantageusement une séparation physique des étanchéités entre, d'une part, le bec de remplissage et la deuxième portion de conduit, et d'autre part, l'embase et la partie fonctionnelle qui est rapportée sur cette embase.
On sépare ainsi avantageusement la zone de remplissage et la zone d'étanchéité de l'assemblage connecteur/partie fonctionnelle pour s'assurer qu'aucune projection de liquide médical survenant éventuellement lors du remplissage du récipient souple, ne puisse devenir une source de moins bonne étanchéité entre la partie fonctionnelle et le connecteur.

L'invention vise aussi un ensemble comprenant une partie fonctionnelle et un connecteur tel que décrit précédemment. Selon l'invention, cette partie fonctionnelle comprend un élément de solidarisation dont la surface externe est destinée à affleurer avec la surface externe de la paroi fermée délimitant l'ouverture de la partie supérieure de l'embase, cette partie fonctionnelle comprenant de plus dans sa partie supérieure une pièce choisie dans le groupe comprenant une tulipe, un bouchon quart de tour sécable, un connecteur luer sécable, un bouchon sécable par le biais d'une zone de rupture annulaire, un site d'injection, ou encore un obturateur tel qu'un « bouchon clou ».

De préférence, ladite embase étant d'un seul tenant et comprenant dans sa partie supérieure une première portion de conduit interne de diamètre D comportant à son extrémité une ouverture, et dans sa partie inférieure, une deuxième portion de conduit interne reliée à ladite première portion de conduit, lesdites première et deuxième portions de conduit étant en communication de fluide, ladite deuxième portion de diamètre d, avec d inférieur à D, formant partiellement saillie à l'intérieur de ladite première portion de manière à définir un logement entre les parois desdites deux portions pour recevoir l'extrémité de ladite partie fonctionnelle, ladite partie fonctionnelle comporte dans sa partie inférieure une jupe dont le diamètre extérieur est inférieur ou égal au diamètre D de ladite première portion de conduit interne de ladite embase, la longueur de ladite jupe étant telle que son extrémité libre est destinée à être au moins partiellement insérée dans ledit logement entre les parois des deux portions de ladite embase, ladite jupe étant alors en communication de fluide avec ladite deuxième portion de conduit interne de ladite embase.

Avantageusement, la surface externe de la jupe et/ou la surface interne de la première portion de conduit interne comportent un ou plusieurs bourrelets pour assurer la tenue mécanique de l'assemblage entre ladite partie fonctionnelle et ladite embase.

La jupe peut encore comprendre au moins une membrane d'étanchéité. Cette membrane peut comporter une ou plusieurs zones prédéterminées d'affaiblissement pour faciliter sa séparation.

L'invention vise enfin un récipient souple pour liquide médical équipé d'au moins un connecteur tel que décrit précédemment et/ou au moins un ensemble tel que décrit précédemment.

L'invention sera décrite plus en détail en référence aux dessins annexés dans lesquels:
- la figure 1 est une vue en perspective d'un ensemble comprenant un connecteur pour récipient souple et une partie fonctionnelle selon un premier mode de réalisation de l'invention;
- la figure 2 montre une vue en perspective du connecteur de la figure 1 ;
- la figure 3 est une vue élargie et en coupe selon l'axe A-A du connecteur de la figure 2 ;
- la figure 4 représente schématiquement un ensemble comprenant un connecteur et une partie fonctionnelle selon un deuxième mode de réalisation de l'invention;
- la figure 5 représente schématiquement un ensemble comprenant un connecteur et une partie fonctionnelle selon un troisième mode de réalisation de l'invention ; la figure 5a) étant une vue en perspective de cet ensemble et la figure 5b) étant une vue en coupe selon l'axe B-B dudit ensemble ;

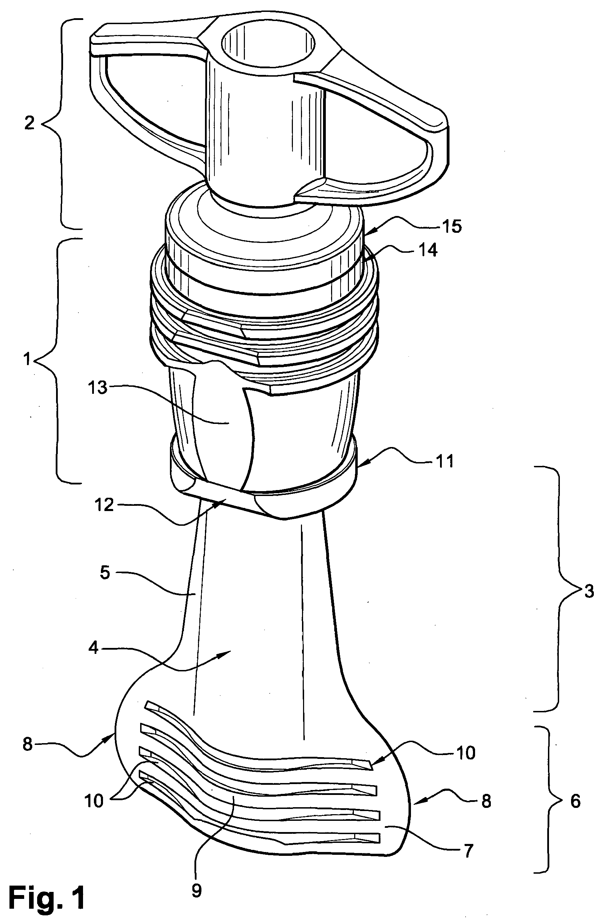

La Figure 1 montre un connecteur médical pour récipient souple auquel est assemblée une partie fonctionnelle comportant à son extrémité un bouchon quart de tour sécable selon un premier mode de réalisation de l'invention.

Le connecteur est d'un seul tenant et obtenu ici par moulage par injection d'une matière plastique médicalement inerte. Il peut, à titre purement illustratif, être réalisé en polycarbonate ou des dérivés de celui-ci, en polyoléfine (tel que le polyéthylène, le polypropylène, ou un copolymère de polypropylène) ou en polystyrène, ou encore en polyoléfine comprenant un thermoplastique élastomère (TPE) tel que le SEB (copolymère bloc Styrène, Ethylène, Butylène), le SEBS (copolymère bloc Styrène, Ethylène, Butylène, Styrène)...

Ce connecteur comprend une embase 1 solidaire à son extrémité supérieure d'une partie fonctionnelle 2. Cette partie fonctionnelle 2 a été rapportée après remplissage du récipient souple avec un liquide médical et éventuellement solidarisée à cette embase 1 par soudure. L'embase 1 est elle même reliée et en communication de fluide à son autre extrémité avec un élément de pincement 3 élastiquement déformable. Cet élément de pincement 3 élastiquement déformable permet avantageusement de clamper le connecteur après remplissage du récipient souple pour empêcher que du liquide médical ne puisse s'échapper de ce récipient durant l'assemblage de la partie fonctionnelle. L'élément de pincement 3 peut être clampé à l'aide d'un organe de clampage tel qu'une pince ou encore manuellement.

Cet élément de pincement 3 élastiquement déformable a une forme de partie tubulaire 4 avec une section transversale 5 divergeant de la forme tubulaire. Cette section transversale 5 présente une forme qui s'évase en s'éloignant de l'embase 1.

Cet élément de pincement 3 est prolongé par un élément de jonction 6 pouvant être intégré dans le récipient souple. Cet élément de jonction comporte sur ses bords latéraux des ailettes 7 formant chacune un prolongement de la partie centrale de cet élément de jonction 6. Ces ailettes 7 sont de plus placées dans le prolongement de la section transversale 5 divergeant de la forme tubulaire de l'élément de pincement 3.

Ces ailettes 7 présentent des bords arrondis 8 au moins dans leur partie supérieure en prolongement direct de la section transversale 5 divergeant de la forme tubulaire. Avantageusement, ces bords arrondis 8 empêchent l'apparition d'un déchirement indésirable au niveau de l'assemblage récipient souple/élément de jonction par manipulation du connecteur. En effet, ces bords arrondis 8, ou incurvés, accroissent le seuil minimal de la force à appliquer pour obtenir un tel déchirement en augmentant la distance de déchirement.

La partie centrale de cet élément de jonction 6 comprend sur les faces supérieure et inférieure de cet élément de jonction, une coupole 9 qui est dans le prolongement de la partie tubulaire 4 de l'élément de pincement 3.

Ces coupoles 9 qui sont alignées, ou sensiblement alignées, sont destinées à coopérer chacune avec un organe presseur creux d'un outil de soudage. Ces coupoles 9 permettent par conséquent d'assurer un auto-positionnement de l'élément de jonction 6 dans ces organes presseur lors de l'assemblage du connecteur avec le récipient souple, l'élément de jonction étant alors pris en sandwich entre les parois du récipient souple.

Pour assurer cet auto-positionnement de l'élément de jonction dans des organes presseurs (moule, contre-moule) de l'outil de soudure qui sont creux et typiquement de coupe ayant une forme bombée telle que semi-circulaire, ces coupoles 9 définissent avantageusement à leur sommet un seul point d'instabilité avec ces organes presseurs et présentent des pentes douces pour assurer un mouvement d'auto-centrage.

Par ailleurs, cet élément de jonction 6 présente des stries de renfort 10 permettant d'assurer un maintien de la forme de la pièce après réalisation de celle-ci.

De préférence, l'embase 1 comporte une collerette 11 comprenant un élément de préhension 12. Cet élément de préhension 12 est ici une découpe permettant l'insertion d'un doigt robotisé pour une manipulation automatisée du connecteur. Cette collerette épaisse 11 est de plus placée sur le connecteur de la Fig.1 à l'extrémité inférieure de l'embase 1 ce qui permet de définir un espace d'approche pour le doigt robotisé puisque l'élément de pincement 3 est placé en retrait de la surface externe de l'embase 1.

L'embase 1 comprend également avantageusement sur sa surface externe deux méplats 13 pour autoriser une préhension manuelle du connecteur. Ces méplats 13 sont ici placés sur les faces supérieure et inférieure de l'embase.

L'embase 1 comporte encore à son extrémité supérieure une ouverture permettant d'introduire dans le volume intérieur de l'embase la partie fonctionnelle 2. Cette ouverture est délimitée par une paroi fermée dont la surface externe 14 est destinée à être placée sensiblement au niveau de la surface externe d'un élément de solidarisation 15 de la partie fonctionnelle lors de l'assemblage du connecteur et de cette partie fonctionnelle pour permettre éventuellement leur soudure.

Par la soudure de ces deux éléments, on s'assure ainsi de la tenue mécanique et de l'étanchéité de la liaison entre le connecteur et la partie fonctionnelle 2. Les risques d'une éventuelle contamination du contenu du récipient souple juste après remplissage de son volume intérieur avec le liquide médical, sont ainsi grandement minimisés.

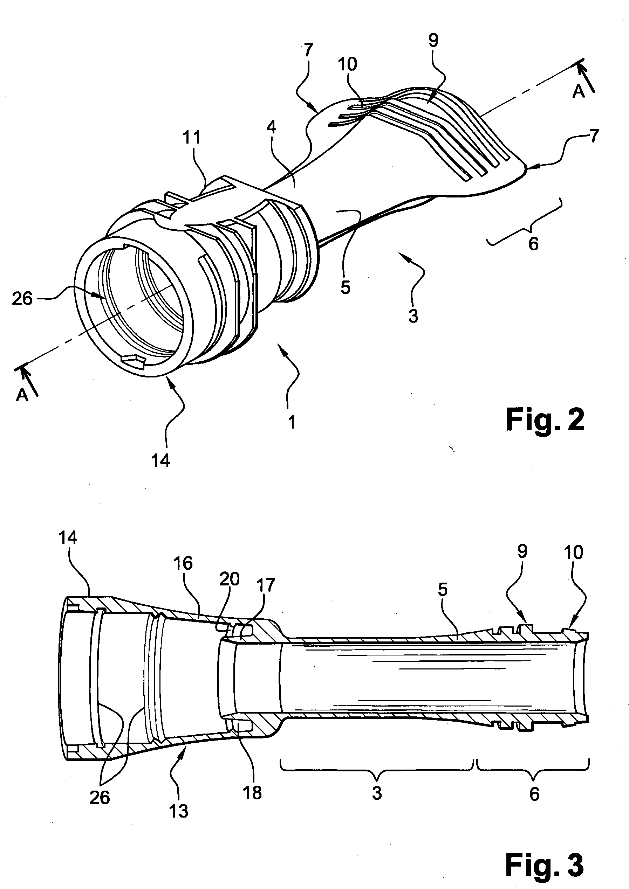

Les Figures 2 et 3 montrent respectivement le connecteur en vue de perspective et en coupe selon l'axe A-A. L'embase 1 comprend dans sa partie supérieure une première portion 16 de conduit interne de diamètre D comportant à son extrémité ladite ouverture destinée à permettre l'introduction de la partie fonctionnelle 2. Elle comporte également, dans sa partie inférieure, une deuxième portion 17 de conduit interne en communication de fluide avec ladite première portion 16 de conduit interne. La première portion de conduit 16 présente donc à son extrémité opposée à celle autorisant l'introduction de la partie fonctionnelle 2, un rétrécissement assurant sa liaison avec la deuxième portion 17 de conduit interne.

Cette deuxième portion 17 de conduit qui a un diamètre d inférieur à D, forme partiellement saillie à l'intérieur de la première portion 16 de manière à définir un logement 18 entre les parois desdites deux portions pour recevoir l'extrémité 19 de la partie fonctionnelle 2.

Un bourrelet continu 20 placé sur la première portion de conduit 16 en vis-à vis de la deuxième portion 17 forme une saillie dans le logement 18 de manière à plaquer l'extrémité 19 de la partie fonctionnelle 2 contre la paroi de la deuxième portion de conduit 17. On s'assure ainsi de l'étanchéité et la tenue mécanique de l'assemblage entre l'embase 1 et la partie fonctionnelle 2.

Avec une réalisation de l'assemblage par soudure de l'embase 1 et de la partie fonctionnelle 2 dans la partie haute de l'embase 1, on obtient ainsi une double protection à la fois en étanchéité et en tenue mécanique de la liaison connecteur/partie fonctionnelle. Cette redondance permet d'être certain qu'au moins une des deux protections fonctionnera en cas de disfonctionnement de l'autre.

La partie de la deuxième portion 17 de conduit interne en saillie dans la première portion 16 de l'embase 1 comporte à son extrémité une paroi amincie 21 par un évidement en forme de tronc de cône inversé. Cette paroi amincie 21 permet de s'assurer de l'étanchéité du contact bec de remplissage/deuxième portion 17 de conduit interne lors du remplissage du récipient souple avec le liquide médical.

La partie fonctionnelle 2 comprend dans sa partie supérieure un bouchon quart de tour 22 sécable par le biais d'une zone de rupture annulaire 23 de ce bouchon avec un élément de solidarisation 15 de la partie fonctionnelle 2. Cet élément de solidarisation 15 a au moins une partie de sa surface externe qui est destinée à affleurer avec la surface externe de la paroi fermée 14 délimitant l'ouverture de l'embase 1 pour autoriser leur solidarisation par soudure.

Le bouchon quart de tour 22 peut être désolidarisé de l'extrémité de la partie fonctionnelle 2 par une simple rotation manuelle d'un quart de tour. Ce bouchon quart de tour 22 permet à l'opérateur de vérifier très aisément, par un simple coup d'oeil, l'intégrité de la substance médicale contenue dans le récipient.

Mais il forme également une double barrière de protection du contenu du récipient souple avec une membrane d'étanchéité placée dans une jupe 25, ou conduit, prolongeant la partie fonctionnelle 2. Cette jupe 25 a un diamètre extérieur Φ inférieur ou égal au diamètre D de la première portion 16 de conduit interne de l'embase 1 de manière à être reçue dans cette portion mais supérieur au diamètre d de la deuxième portion 17 de conduit de manière que son extrémité soit au moins partiellement reçue dans le logement 18. Une fois placée en position, cette jupe 25 est en communication de fluide avec la deuxième portion 17 de conduit interne de l'embase 1.

La surface intérieure de la première portion 16 de conduit comporte de préférence un ou plusieurs bourrelets 26 pour assurer la tenue mécanique de l'assemblage entre la partie fonctionnelle 2 et l'embase 1. Alternativement, ce ou ces bourrelets 26 peuvent également être placés sur la surface externe de la jupe 25, ou encore, à la fois sur la surface intérieure de la première portion 16 de conduit et sur la surface externe de la jupe 25.

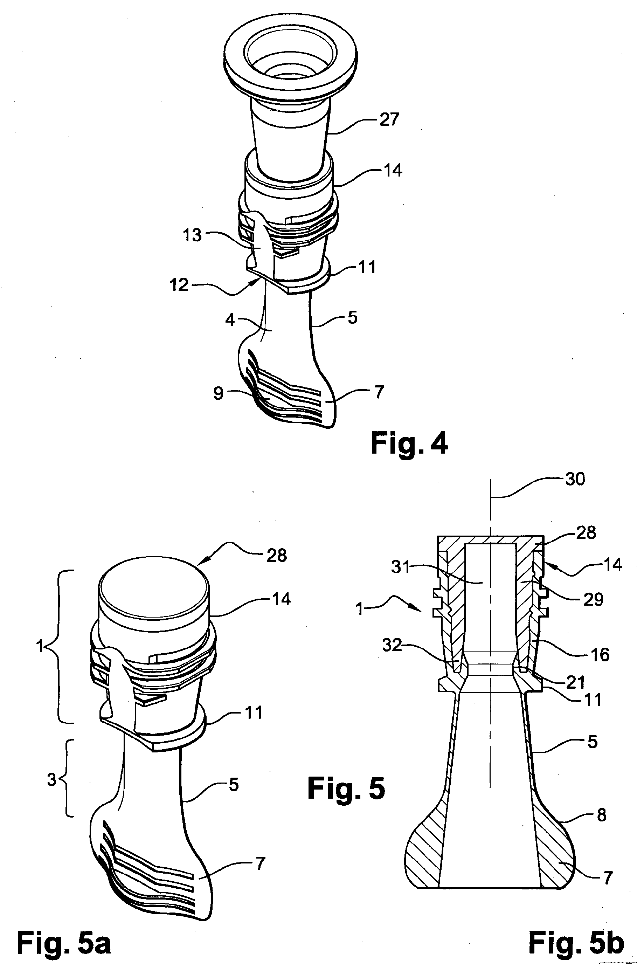

La Figure 4 montre un ensemble comprenant un connecteur tel que décrit précédemment et une partie fonctionnelle selon un deuxième mode de réalisation de l'invention. Cette partie fonctionnelle comporte un élément évasé 27 qui est communément appelé une tulipe en raison de la forme de cet élément évasé. Cette tulipe 27 permet de recevoir l'extrémité d'un flacon pour assurer le mélange du contenu de ce flacon avec le contenu du volume intérieur du récipient tel qu'une poche souple, sur lequel est monté cet ensemble.

La figure 5 montre un ensemble comprenant un connecteur tel que décrit précédemment et une partie fonctionnelle qui est ici un obturateur tel qu'un « bouchon clou ». La figure 5b) montre une vue en coupe selon l'axe B-B de l'ensemble de la figure 5a).

Cet obturateur d'un seul tenant comporte un bouchon 28 dont le pourtour affleure avec la surface externe de la paroi fermée 14 délimitant l'ouverture de l'embase 1 pour autoriser leur éventuelle solidarisation par soudure. Ce bouchon 28 est prolongé par une jupe 29 ayant une épaisseur variable le long de son axe de symétrie principal 30 de manière à conserver un canal interne 31 de diamètre constant. De plus, sa partie inférieure présente une paroi amincie 32 par un évidement en forme de tronc de cône de sorte que l'extrémité libre de cette jupe 29 coopère avec le logement 18 de l'embase 1 pour assurer l'assemblage étanche de l'obturateur et de cette embase 1.

## Revendications

1. Connecteur pour récipient souple destiné à contenir un liquide médical, ledit connecteur comprenant un conduit interne pour le passage du liquide et une embase (1) destinée à recevoir, à une de ses extrémités, une partie fonctionnelle (2) dudit connecteur, ladite embase (1) étant reliée et en communication de fluide à son autre extrémité avec un élément de pincement (3) élastiquement déformable, ledit élément élastiquement déformable étant prolongé par un élément de jonction (6) qui peut être intégré dans ledit récipient souple, ledit élément de jonction (6) comportant à chacun de ses bords latéraux une ailette, lesdites ailettes (7) formant chacune un prolongement de la partie centrale dudit élément de jonction (6),
**caractérisé en ce que**
- la partie centrale dudit élément de jonction (6) comprend sur chacune des faces supérieure et inférieure dudit élément de jonction (6), une au moins demi-coupole (9) placée à l'extrémité dudit élément de jonction (6) en formant saillie du reste de la face correspondante dudit élément de jonction, ladite au moins une demi-coupole (9) définissant à son sommet un seul point d'instabilité_avec un organe presseur creux d'un outil de soudage pour assurer un auto-positionnement dudit élément de jonction (6) dans ledit organe presseur,
- ledit élément de pincement (3) élastiquement déformable ayant une forme de partie tubulaire avec une section transversale divergeant (5) de la forme tubulaire, lesdites ailettes (7) sont placées dans le prolongement de ladite section transversale divergeant (5) de la forme tubulaire, et **en ce que**
- ladite une au moins demie coupole (9) est dans le prolongement de la partie tubulaire dudit élément de pincement (3).

2. Connecteur selon la revendication 1, **caractérisé en ce que** lesdites ailettes (7) comprennent des bords arrondis (8) au moins dans leur partie supérieure en prolongement de ladite section transversale divergeant (5) de la forme tubulaire.

3. Connecteur selon la revendication 1 ou 2, **caractérisé en ce que** ladite partie centrale comprend sur chacune des faces supérieure et inférieure dudit élément de jonction (6), une coupole (9).

4. Connecteur selon l'une quelconque des revendications 1 à 3, **caractérisé en ce que** ladite embase (1) comprend à l'extrémité de sa partie supérieure une ouverture délimitée par une paroi fermée dont la surface externe (14) est destinée à être placée sensiblement au niveau de la surface externe d'un élément de solidarisation (15) de ladite partie fonctionnelle (2) pour permettre leur soudure.

5. Connecteur selon l'une quelconque des revendications 1 à 4, **caractérisé en ce que** ladite embase (1) étant d'un seul tenant, elle comporte dans sa partie supérieure une première portion (16) de conduit interne de diamètre D comportant à son extrémité une ouverture, et dans sa partie inférieure, une deuxième portion (17) de conduit interne reliée à ladite première portion de conduit, lesdites première et deuxième portions de conduit (16, 17) étant en communication de fluide, ladite deuxième portion (17) de diamètre d, avec d inférieur à D, formant partiellement saillie à l'intérieur de ladite première portion de manière à définir un logement (18) entre les parois desdites deux portions pour recevoir l'extrémité de ladite partie fonctionnelle (2).

6. Connecteur selon la revendication 5, **caractérisé en ce que** ladite première portion (16) comporte un bourrelet continu (20) placé en vis-à vis de ladite deuxième portion (17) en saillie dans ledit logement pour assurer l'étanchéité et la tenue mécanique de l'assemblage entre ladite embase (1) et ladite partie fonctionnelle (2).

7. Connecteur selon la revendication 5 ou 6, **caractérisé en ce que** la partie de ladite deuxième portion en saillie dans ladite première portion de l'embase (1) comporte à son extrémité une paroi amincie (21) par un évidement en forme de tronc de cône inversé pour assurer l'étanchéité avec un bec de remplissage.

8. Connecteur selon l'une quelconque des revendications 1 à 7, **caractérisé en ce que** ladite embase (1) comporte une collerette (11) comprenant un élément de préhension (12).

9. Connecteur selon l'une quelconque des revendications 1 à 8, **caractérisé en ce que** ladite embase (1) comprend sur sa surface externe deux méplats (13) pour autoriser une préhension manuelle dudit connecteur.

10. Ensemble comprenant une partie fonctionnelle (2) et un connecteur selon l'une quelconque des revendications 1 à 9, **caractérisé en ce que** ladite partie fonctionnelle (2) comprend un élément de solidarisation dont la surface externe est destinée à affleurer avec la surface externe de la paroi fermée délimitant ladite ouverture de la partie supérieure de ladite embase (1), ladite partie fonctionnelle (2) comprenant de plus dans sa partie supérieure une pièce choisie dans le groupe comprenant une tulipe, un bouchon quart de tour sécable, un connecteur luer sécable, un bouchon sécable par le biais d'une zone de rupture annulaire ou encore un site d'injection.

11. Ensemble selon la revendication 10, **caractérisé en ce que** ledit connecteur est un connecteur selon l'une quelconque des revendications 1 à 9 et 5 et **en ce que** ladite partie fonctionnelle (2) comporte dans sa partie inférieure une jupe (25) dont le diamètre extérieur est inférieur ou égal au diamètre D de ladite première portion (16) de conduit interne de ladite embase (1), la longueur de ladite jupe (25) étant telle que son extrémité libre est destinée à être au moins partiellement insérée dans ledit logement entre les parois des deux portions de ladite embase (1), ladite jupe (25) étant alors en communication de fluide avec ladite deuxième portion (17) de conduit interne de ladite embase (1).

12. Ensemble selon la revendication 11, **caractérisé en ce que** la surface externe de ladite jupe (25) et/ou la surface interne de ladite première portion (16) de conduit interne comportent un ou plusieurs bourrelets (26) pour assurer la tenue mécanique de l'assemblage entre ladite partie fonctionnelle (2) et ladite embase (1).

13. Ensemble selon la revendication 11 ou 12, **caractérisé en ce que** ladite jupe (25) comprend au moins une membrane d'étanchéité.

14. Récipient souple pour liquide médical équipé d'au moins un connecteur selon l'une quelconque des revendications 1 à 9 et/ou au moins un ensemble selon l'une des revendications 10 à 13.

## Patentansprüche

1. Verbinder für einen flexiblen Behälter, der dazu bestimmt ist, eine medizinische Flüssigkeit zu enthalten, wobei der Verbinder einen inneren Kanal für den Durchhang der Flüssigkeit und einen Ansatz (1) umfasst, der dazu bestimmt ist, an einem seiner Enden ein Funktionsteil (2) des Verbinders aufzunehmen, wobei der Ansatz (1) an seinem anderen Ende mit einem elastisch deformierbaren Kneifelement (3) verbunden ist und damit in Fluidkommunikation steht, wobei sich das elastisch deformierbare Element in einem Anschlusselement (6) fortsetzt, das in den flexiblen Behälter integriert sein kann, wobei das Anschlusselement (6) an jedem seiner Seitenränder eine Finne umfasst, wobei die Finnen (7) jeweils eine Fortsetzung des mittleren Teils des Anschlusselements (6) bilden, dadurch gekennzeichet, dass
- der mittlere Teil des Anschlusselements (6) an der oberen und der unteren Fläche des Anschlusselements (6) jeweils mindestens eine Halbkuppel (9) umfasst, die am Ende des Anschlusselements (6) platziert ist und einen Vorsprung vom Rest der entsprechenden Fläche des Anschlusselements bildet, wobei die mindestens eine Halbkuppel (9) an ihrem Gipfel eine einzige Instabilitätsstelle mit einem hohlen Druckorgan eines Schweißwerkzeugs definiert, um eine automatische Positionierung des Anschlusselements (6) im Druckorgan zu gewährleisten,
- die Finnen (7) - wobei das elastisch deformierbare Kneifelement (3) die Form eines röhrenförmigen Teils mit einem von der Röhrenform divergierenden Querschnitt (5) hat - in der Fortsetzung des von der Röhrenform divergierenden Querschnitts (5) platziert sind, und dass
- die mindestens eine Halbkuppel (9) in der Fortsetzung des röhrenförmige Teils des Kneifelements (3) liegt.

2. Verbinder nach Anspruch 1, **dadurch gekennzeichnet, dass** die Finnen (7) mindestens an ihrem oberen Teil, der den von der Röhrenform divergierenden Querschnitt (5) fortsetzt, abgerundete Ränder (8) umfassen.

3. Verbinder nach Anspruch 1 oder 2, **dadurch gekennzeichnet, dass** der mittlere Teil an der oberen und unteren Fläche des Anschlusselements (6) jeweils eine Kuppel (9) umfasst.

4. Verbinder nach einem der Ansprüche 1 bis 3, **dadurch gekennzeichnet, dass** der Ansatz (1) am Ende seines oberen Teils eine Öffnung umfasst, die durch eine geschlossene Wand begrenzt ist, deren Außenfläche (14) dazu bestimmt ist, im Wesentlichen auf der Höhe der Außenfläche eines Verbindungselements (15) des Funktionsteile (2) platziert zu werden, um ihr Verschweißen zu gestatten.

5. Verbinder nach einem der Ansprüche 1 bis 4, **dadurch gekennzeichnet, dass** der Ansatz (1) einteilig ist und an seinem oberen Teil einen ersten Innenkanalabschnitt (16) mit einem Durchmesser D, der an seinem Ende eine Öffnung umfasst, und an seinem unteren Teil einen zweiten Innenkanalabschnitt (17) umfasst, der mit dem ersten Kanalabschnitt verbunden ist, wobei der erste und der zweite Kanalabschnitt (16, 17) in Fluidkommunikation stehen, wobei der zweite Abschnitt (17) mit Durchmesser d, wobei d kleiner als D ist, teilweise in das Innere des ersten Abschnitts vorragt, um eine Aufnahme (18) zwischen den Wänden der beiden Abschnitte zu definieren, um das Ende des Funktionsteils (2) aufzunehmen.

6. Verbinder nach Anspruch 5, **dadurch gekennzeichnet, dass** der erste Abschnitt (16) einen durchgehenden Wulst (20) umfasst, der dem zweiten Abschnitt (17) gegenüber in die Aufnahme vorragend platziert ist, um die Dichtheit und die mechanische Festigkeit der Montage zwischen dem Ansatz (1) und dem Funktionsteil (2) zu gewährleisten.

7. Verbinder nach Anspruch 5 oder 6, **dadurch gekennzeichnet, dass** der Teil des zweiten Abschnitts, der in den ersten Abschnitt des Ansatzes (1) vorragt, an seinem Ende eine Wand (21) umfasst, die durch eine umgekehrt kegelstumpfförmige Aussparung verdünnt ist, um die Dichtheit mit einem Füllmundstück zu gewährleisten.

8. Verbinder nach einem der Ansprüche 1 bis 7, **dadurch gekennzeichnet, dass** der Ansatz (1) einen Bund (11) umfasst, der ein Greifelement (12) umfasst.

9. Verbinder nach einem der Ansprüche 1 bis 8, **dadurch gekennzeichnet, dass** der Ansatz (1) an seiner Außenfläche zwei Abflachungen (13) umfasst, um ein manuelles Ergreifen des Verbinders zu gestatten.

10. Anordnung, umfassend ein Funktionsteil (2) und einen Verbinder nach einem der Ansprüche 1 bis 9, **dadurch gekennzeichnet, dass** das Funktionsteil (2) ein Verbindungselement umfasst, dessen Außenfläche dazu bestimmt ist, mit der Außenfläche der geschlossenen Wand, die die Öffnung des oberen Teils des Ansatzes (1) begrenzt, bündig zu sein, wobei das Funktionsteil (2) darüber hinaus an seinem oberen Teil eine Komponente umfasst, die aus der eine Muffe, einen zerbrechbaren Vierteldrehungsstopfen, einen zerbrechbaren Luer-Verbinder, einen mittels einer ringförmigen Brechzone zerbrechbaren Stopfen oder eine Injektionsstelle umfassenden Gruppe ausgewählt ist.

11. Anordnung nach Anspruch 10, **dadurch gekennzeichnet, dass** der Verbinder ein Verbinder nach einem der Ansprüche 1 bis 9 und 5 ist und dass das Funktionsteil (2) an seinem unteren Teil einen Schurz (25) umfasst, dessen äußerer Durchmesser kleiner oder gleich dem Durchmesser D des ersten Innenkanalabschnitts (16) des Ansatzes (1) ist, wobei die Länge des Schurzes (25) derart ist, dass sein freies Ende dazu bestimmt ist, mindestens teilweise in die Aufnahme zwischen den Wänden der beiden Abschnitte des Ansatzes (1) eingeführt zu werden, wobei der Schurz (25) somit in Fluidkommunikation mit dem zweiten Innenkanalabschnitt (17) des Ansatzes (1) steht.

12. Anordnung nach Anspruch 11, **dadurch gekennzeichnet, dass** die Außenfläche des Schurzes (25) und/oder die Innenfläche des ersten Innenkanalabschnitts (16) einen oder mehrere Wülste (26) umfassen, um die mechanische Festigkeit der Montage zwischen dem Funktionsteil (2) und dem Ansatz (1) zu gewährleisten.

13. Anordnung nach Anspruch 11 oder 12, **dadurch gekennzeichnet, dass** der Schurz (25) mindestens eine Dichtmembran umfasst.

14. Flexibler Behälter für eine medizinische Flüssigkeit, der mit mindestens einem Verbinder nach einem der Ansprüche 1 bis 9 und/oder mindestens einer Anordnung nach einem der Ansprüche 10 bis 13 ausgerüstet ist.

## Claims

1. Connector for a flexible container for receiving a medical liquid, said connector comprising an inner conduit for the passage of the liquid and a collar (1) for receiving, at one of its ends, a functional part (2) of said connector, said collar (1), at its other end, being connected to and in fluid communication with an elastically deformable pinching element (3), said elastically deformable element being continued by a junction element (6) that can be integrated in said flexible container, said junction element (6) having a fin at each of its lateral edges, said fins (7) each forming a continuation of the central part of said junction element (6),
**characterized in that**
- the central part of said junction element (6) comprises, on each of the upper and lower faces of said junction element (6), at least one half-dome (9) placed at the end of said junction element (6) and forming a projection from the rest of the corresponding face of said junction element, said at least one half-dome (9) defining, at its summit, a single point of instability with a hollow pressing member of a welding tool in order to ensure self-positioning of said junction element (6) in said pressing member,
- said elastically deformable pinching element (3) having the shape of a tubular part with a cross section diverging (5) from the tubular shape, said fins (7) are placed in the continuation of said cross section diverging (5) from the tubular shape, and **in that**
- said at least one half-dome (9) is in the continuation of the tubular part of said pinching element (3).

2. Connector according to Claim 1, **characterized in that** said fins (7) comprise rounded edges (8) at least in their upper part that continues said cross section diverging (5) from the tubular shape.

3. Connector according to Claim 1 or 2, **characterized in that** said central part comprises a dome (9) on each of the upper and lower faces of said junction element (6).

4. Connector according to any one of Claims 1 to 3, **characterized in that** said collar (1) comprises, at the end of its upper part, an opening delimited by a closed wall whose outer surface (14) is intended to be placed substantially level with the outer surface of a securing element (15) of said functional part (2) in order to permit their welding.

5. Connector according to any one of Claims 1 to 4, **characterized in that** said collar (1) is in one piece and has, in its upper part, a first inner conduit portion (16) of diameter D with an opening at its end, and, in its lower part, a second inner conduit portion (17) connected to said first conduit portion, said first and second conduit portions (16, 17) being in fluid communication, said second portion (17) of diameter d, with d smaller than D, partially projecting inside said first portion in such a way as to define a seat (18) between the walls of said two portions for receiving the end of said functional part (2).

6. Connector according to Claim 5, **characterized in that** said first portion (16) has a continuous bead (20) placed opposite said second portion (17) projecting into said seat in order to ensure the leaktightness and mechanical strength of the join between said collar (1) and said functional part (2).

7. Connector according to Claim 5 or 6, **characterized in that** the part of said second portion projecting into said first portion of the collar (1) has, at its end, a wall (21) that is made thin by a recess in the shape of an inverted truncated cone in order to ensure the leaktightness with a filling spout.

8. Connector according to any one of Claims 1 to 7, **characterized in that** said collar (1) has a flange (11) comprising a gripping element (12).

9. Connector according to any one of Claims 1 to 8, **characterized in that** said collar (1) comprises two flat parts (13) on its outer surface, in order to permit manual gripping of said connector.

10. Assembly comprising a functional part (2) and a connector according to any one of Claims 1 to 9, **characterized in that** said functional part (2) comprises a securing element, of which the outer surface is intended to be flush with the outer surface of the closed wall delimiting said opening of the upper part of said collar (1), said functional part (2) moreover comprising, in its upper part, a component chosen from the group including a bell, a breakable quarter-turn stopper, a breakable Luer connector, a stopper breakable by way of an annular tear zone, or an injection site.

11. Assembly according to Claim 10, **characterized in that** said connector is a connector according to any one of Claims 1 to 9 and 5 and **in that** said functional part (2) has, in its lower part, a skirt (25) whose external diameter is less than or equal to the diameter D of said first inner conduit portion (16) of said collar (1), the length of said skirt (25) being such that its free end is intended to be inserted at least partially into said seat between the walls of the two portions of said collar (1), said skirt (25) then being in fluid communication with said second inner conduit portion (17) of said collar (1).

12. Assembly according to Claim 11, **characterized in that** the outer surface of said skirt (25) and/or the inner surface of said first inner conduit portion (16) have one or more beads (26) for ensuring the mechanical strength of the join between said functional part (2) and said collar (1).

13. Assembly according to Claim 11 or 12, **characterized in that** said skirt (25) comprises at least one sealing membrane.

14. Flexible container for medical liquid, equipped with at least one connector according to any one of Claims 1 to 9 and/or at least one assembly according to one of Claims 10 to 13.
